# EUROPEAN PATENT APPLICATION

(11) **EP 2 781 204 A1**
(43) Date of publication of application: **24.09.2014**
(21) Application number: 14000996.0
(22) Date of filing: 18.03.2014
(51) Int. Cl.: A61F 2/07, A61B 17/11, A61F 2/06, A61F 2/958, A61F 2/848

(54) **Intravascular system for introducing and fastening an autogenous vascular prosthesis**

(30) Priority: 19.03.2013 PL 40322013
(71) Applicant: BALTON Spolka z ograniczona odpowiedzialnoscia, 00-496 Warszawa (PL)
(72) Inventor: Plowiecki, Emil, 00-496 Warszawa (PL); Hurkala, Leszek, 00-496 Warszawa (PL)
(74) Representative: Gizinska-Schohe, Malgorzata

(57) **Abstract**

The invention relates to an intravascular system for introducing and attaching an autogenous vascular prosthesis, comprising two stents, characterised in that it comprises a proximal stent **(1)** and a distal stent **(3),** located one behind the other, each of the stents being provided with at least two suture sections **(5),** attached to the stents, free ends of the said sections being located outside the lumen of the stents. The invention also relates to a method of attaching an autogenous vascular prosthesis.

## Description

The object of the invention is an intravascular system for introducing and attaching an autogenous vascular prosthesis and a method of attaching the autogenous vascular prosthesis. The invention relates to the field of medical devices - intravascular catheters - intended for precisely introducing, and then positioning and installing, in the treated blood vessel, a configuration of stents which constitute a structural support for an autogenous vascular prosthesis, the device being particularly intended for the treatment of vascular disease conditions, such as aneurysms, especially occurring in the area of peripheral arteries, e.g. popliteal arteries.

Currently, in addition to synthetic materials known for many years and used in the reconstruction of blood vessels, biological material are also used. Autogenous vascular grafts (autogenous material) are the elements made from the patient's own tissue; usually, in vascular surgery, they are veins collected from the patient directly before the actual procedure of reconstructing blood vessels. In the presented solution, the use of an autogenous prosthesis is presented.

Aneurysms of popliteal artery constitute about 75% of aneurysms of peripheral arteries. Their etiology is in 95% of atherosclerotic nature, which may additionally cause complications in surgical treatment which is the primary method of treatment of popliteal artery aneurysm. The surgery consists in removing the region of the modified artery or its ligation, or clipping, and restoring blood flow through implantation of an insert at this place, or in performing a bypass graft. The synthetic material most commonly used in the preparation of vascular prostheses, e.g. stentgrafts, is polytetrafluoroethylene or polyester; however, better results are obtained when applying a vascular insert from a vein collected from the patient (autogenous prosthesis). However, the described surgical reconstruction of blood vessels is an invasive method, can cause complications and is especially inadvisable for the most vulnerable group of patients. i.e. elderly patients who additionally have coexisting pathological syndromes.

Vascular aneurysms are also treated with a less invasive method, through transdermal methods, e.g. by introducing, through the femoral artery, intravascular catheters comprising appropriate stents or stentgrafts installed at the location of aneurysm occurrence.

From US patent specifications US 5,316,023, US 5,360,443, US 5,578,071 and US 5,591,229, systems for transdermal, intravascular introduction of tubular structures at the location of aneurysm occurrence are known. The mentioned structures (prostheses) for the reconstruction of damaged blood vessels can be made based on polyesters (e.g. Dacron®), polytetrafluoroethylene (e.g. Teflon®), porous polyurethane, or silicone. These structures are most often installed and stabilised by supporting structural elements, such as stents which are most often located at the ends of such tubular prostheses. Stents can be attached outside or inside prostheses. Usually, these stents are mounted on an expandable balloon (system based on a balloon catheter) but they can be also self-expanding stents, e.g. made of nitinol.

For example, from Polish patent specification PL190012B1 (International Application WO9929262), an intravascular aortic prosthesis, intended in particular for the treatment of infrarenal aortic aneurysm, is known. The prosthesis is composed of at least two Z-shaped stents, made of stainless steel or nitinol, which are sutured to a tube of biocompatible material. The prosthesis is installed by releasing it from the delivery device transdermally introduced into the body of the patient, through the femoral artery in its inguinal section. Compression of the prosthesis before the surgery, and then its opening, once introduced into the vessel, are allowed by actuating wires.

Introduction of these less invasive methods of treatment made it possible to simplify surgeries and reduced the risk of postsurgical complications, and consequently the mortality rate has decreased. However, for specific aneurysms, such as popliteal aneurysms, the above methods have many limitations as satisfactory results are not always obtained due to mechanical factors. Therefore, there is a need to further improve the above methods and to adapt them to the knee joint which is a characteristic region of the body.

The object of the invention is an intravascular system for introducing and attaching an autogenous vascular prosthesis, comprising two stents, characterised in that it comprises a proximal stent and a distal stent, located one behind the other, each stent being provided with at least two suture sections, attached to the stents, free ends of the said sections being located outside the lumen of the stents.

Preferably, the proximal stent is provided with two or three, or four suture sections, and the distal stent is provided with two or three, or four suture sections.

Preferably, the suture sections attached to one stent are located at the same distance from the edge of this stent, wherein distances of suture attachment in the proximal and distal stent can be the same or different.

Preferably, the suture sections are attached on the circumference of the stent at equal distances to each other.

Preferably, the suture sections are attached to the stents at a distance comprised in the range of 25% to 75% of the length of the stents.

Preferably, the stents comprised in the system are self-expanding stents and/or balloon-expandable stents.

The object of the invention is also a method of attaching an autogenous vascular prosthesis, especially at the location of aneurysm occurrence within the knee joint, characterised in that a vein section collected from the patient, overlaps extracorporeally the intravascular system comprising a proximal stent and a distal stent, located one behind the other, each stent being provided with at least two suture sections, attached to the stents, so that the system is located in the lumen of the implanted vein, and then, still extracorporeally, by free ends of the suture sections located outside the lumen of the stents, the proximal edge of the vein is sutured to the proximal stent, on its circumference, and the distal edge of the vein is sutured to the distal stent, on its circumference, superfluous suture sections are removed, then the system comprising the prepared autogenous prosthesis is placed in a shielding tube and is introduced intravascularly to the location of aneurysm occurrence, the prosthesis is placed in position in the section of aneurysm occurrence, and the proximal and distal stents of the prosthesis are attached sequentially or simultaneously, and the entire autogenous prosthesis is attached in the vessel by releasing it from the system.

The system according to the invention enables attaching, in a blood vessel, especially at the location of aneurysm occurrence within the knee joint, an autogenous prosthesis (internal "by-pass") which is constituted by a configuration of two stents between which a portion of the vein collected from the patient is attached.

Having collected the vein, the doctor can, beyond the patient's body, quickly and precisely attach the collected vein to a system specially adapted for this purpose, and then he can introduce the entire system together with the vein into the vessel in order to attach the prosthesis inside the vessel. The system ensures a safe procedure of attaching the vein to the system as the risk of damaging the system components at the time of suturing the vein section to the stents is minimised. In particular, risky manipulation with the surgical needle is minimised. This is important when using balloon catheters since the stents to which a vein section is sutured are clamped on an expandable balloon, the damage of which with the surgical needle would prevent inflating it in the next step and consequently attaching the stent inside the vessel. Safety of the vein suturing procedure is reached thanks to the fact that the stents comprised in the system are provided with ready suture sections attached to them. The sutures are braided (attached) precisely on the stents, before placing them in the system, i.e. before clamping them on balloons. This allows fast, easy and safe suturing of the vein collected from the patient so that the vein section together with the stents constitute a combined entirety - a vascular prosthesis. The doctor sutures one end of the collected vein section to one stent in its central part, and the other end of the vein section to the other stent, preferably also in the central part of the stent. Braiding the sutures through eyelets of the stents clamped on the balloon is eliminated as the sutures are already attached on the stent. The doctor uses suture sections by attaching them only to end edges of the vein.

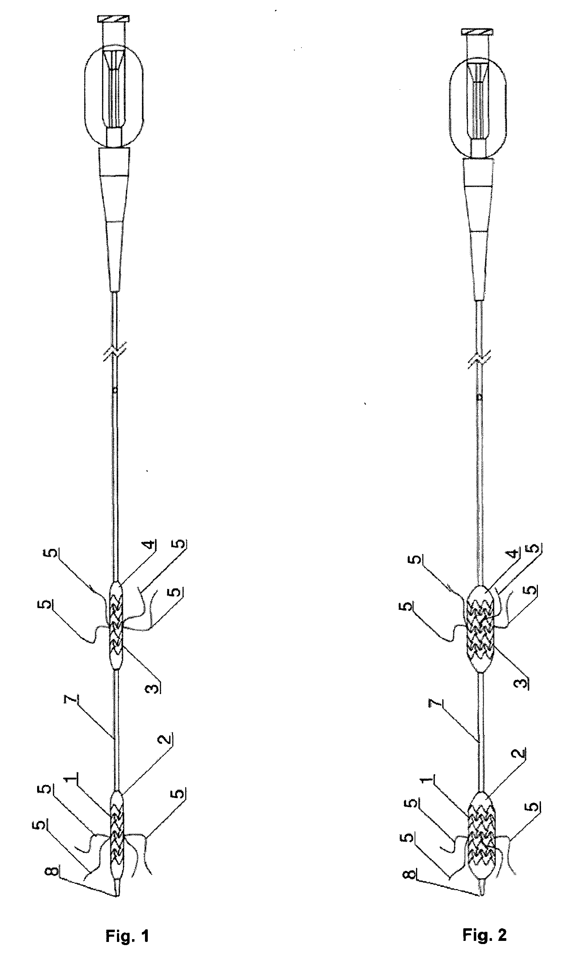

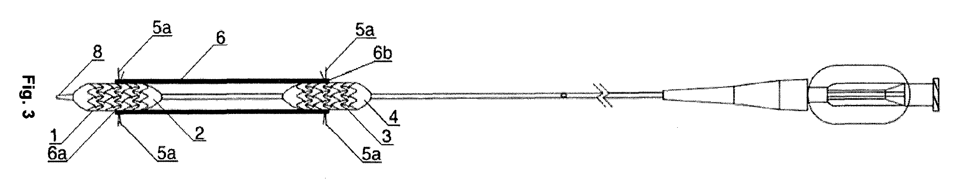

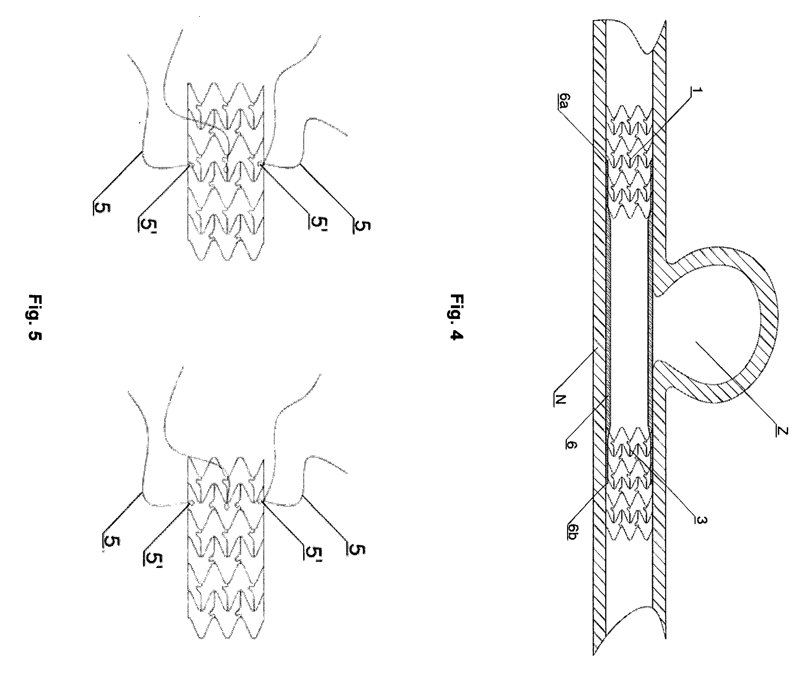

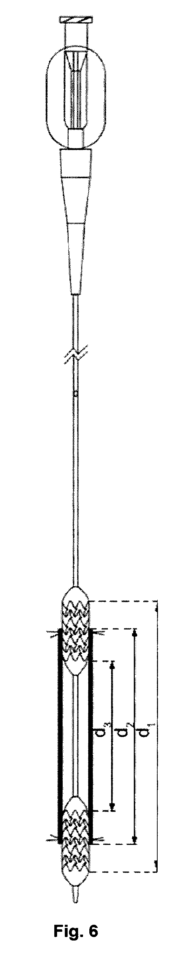

The object of the invention in an exemplary embodiment is illustrated in the drawing, in which Fig. 1 shows an overall view of the system for implanting an autogenous prosthesis, Fig. 2 shows the system of Fig. 1, wherein the system is shown with balloons partially inflated for better visibility of the stents, Fig. 3 shows the system after suturing the vein section to the stents, Fig. 4 shows a prosthesis composed of two stents and a vein section, attached in the lumen of a pathologically changed vessel, Fig. 5 shows stents provided with suture sections in two variants; on the left, suture sections are attached in half the length of the stent, and on the right, a variant in which suture sections are attached in 1/3 of the length of the stent is shown, and Fig. 6 shows the system of Fig. 3 with distances d₁, d₂ and d₃ indicated.

Intravascular system for introducing and attaching an autogenous vascular prosthesis according to the invention comprises a configuration of two stents arranged in series, one after the other - a proximal stent 1 and a distal stent 3, both of them being provided with at least two suture sections 5 attached on the circumference of the stents to stent eyelets by means of retaining loops 5'. In case of a system based on balloon-expandable stents, the proximal stent 1 is mounted on a proximal balloon **2**, and the distal stent **3** is mounted on a distal balloon **4.** Having sutured a vein section **6** to the configuration of two stents, the system comprises an autogenous prosthesis composed of two stents **(1, 3)** and the vein section **6** attached between them. A proximal edge **6a** of the vein section is attached to the proximal stent **1,** and a distal edge **6b** of the vein section is attached to the distal stent. Having sutured the vein section **6** to the stents, suture sections **5** are cut off (removal of superfluous suture portions) and suture ends **5a** remain visible on the combined vascular prosthesis.

The intravascular system for attaching the prosthesis also comprises typical elements which are easy to predict by the skilled person, such as a tip **8** which is the first to be introduced into the patient's body and is appropriately profiled and made of a soft material. The system also comprises guide channels and balloon inflation/deflation channels, and an external tube **7** to which balloons with the stents mounted are attached. The system also comprises appropriate handles and caps which allow handling the system, and shielding tubes for the systems. The system can be also connected to typical devices which control balloon inflation and pressures reached in balloons.

Intravascular systems, known in the field of angioplasty, e.g. rapid exchange (RX) systems based on the rapid exchange of the catheter without any need to introduce the guide and over-the-wire systems, can be used for the purposes of the invention.

In case of using the balloon catheter, it may be configured so that the balloon inflation is accomplished simultaneously or separately for each balloon. It will be apparent that in systems of these types, a customised configuration of channels for inflating balloons is used.

Suture sections are located in at least two, preferably three or four places on the circumference of the stent, wherein the said two, three or four suture sections are located in a specific, equal distance from the edge of the stent so that the end edge of the vein section overlapping the stent could be sutured in two, three or four places on its circumference. E.g. the sutures can be attached in two places on the circumference of the stent, oppositely, i.e. in a position at a distance of 180° relative to each other or in three places on the circumference of the stent, every 120°, or in four places, every 90°. At the same time, these places of attachment of suture sections are preferably located at equal distances from the proximal and distal edges of the stent, i.e. in half the length of the stent.

In such an embodiment of the invention, the vein section collected from the patient and properly prepared will be applied to both stents so that it constitutes, at this point, an external element on the system - in the lumen of the vein, the both stents and other structural elements of the system will be partially present. In the described embodiment, the applied vein section partially covers the stent ends, which face each other, so that the vein edges are located in half the length of the stents where suture sections are attached. Outer (distant from the vein section) portions of the stents remain uncovered by the vein collected from the patient. Once implanted, these uncovered parts of the stents will directly adhere to the walls of the vessel against which the implanted portion of the vein is pressed.

Such an embodiment of the invention is shown in Fig. 1 (before suturing the vein) and in Fig. 3 (after suturing the vein), and in Fig. 4 (after implantation of the prosthesis in the lumen of the vessel). In this embodiment, suture sections are attached to both stents in half their lengths. The stents are provided with two suture sections located on the circumference of the stent at distances of 180° relative to each other. The sections are attached to the stents (tied in the stent eyelets) in the middle of their length - thereby, two ends available for use by the doctor will be formed from each one of the suture sections. Fig. 3 shows that in such an embodiment of the system according to the invention, the vein section is sutured in half the length of the stents. Thereby, outer - in relation to the attached vein section - halves of the stents remain uncovered, and the stent parts which face each other are covered by the vein section which is slipped onto them. Fig. 4 shows how, in such an embodiment of the invention, the stent attachment in a pathologically changed vessel will look like. The uncovered outer halves of the stents directly adhere to the walls of the vessel, and the inner parts, which face each other, of the stents are covered by the sutured vein section and press this vein section, on its both ends, against the walls of the vessel.

In another embodiment of the invention, places for attaching sutures can be located at a distance equal to 1/3 of the length of the stent (Fig. 5), wherein it is possible to arrange the stents in the system so that 1/3 of the length of both stents will be covered by the vein section applied on the system, or so that 2/3 of the length of both stents will be located inside the vein section applied on them.

It is also possible to have such an embodiment of the system according to the invention that each of the stents, on which the vein section is mounted, is provided with sutures located at different locations on the stent, i.e. one stent comprises suture sections mounted in half its length, and the other in one-third of its length. Location of sutures determines the place for attaching the edges of the implemented vein section - i.e. in the described case, on one side of the vein, half of the stent will be in its interior, and on the other side, 1/3 of the stent will be in its interior. The remaining, free portions of the stent will be pressed directly against the vessel wall, after installing the prosthesis.

The place for attaching the suture sections on the circumference of the stent should be spaced from its free (uncovered by the implanted vein section) edge at least by 10% of its length, preferably 25% of its length, more preferably 50% of its length. The sutures can be attached preferably at a distance of 25 to 75% of the stent length, starting from its uncovered edge. In the mentioned embodiments of the system, portions of the stent, which amount to respectively at least 10% of its length, 25% of its length, 50% of its length, preferably comprised in the range of 25% to 75%, will remain uncovered.

Fig. 6 shows a system comprising an autogenous prosthesis constituting a configuration of two stents with a vein section sutured to them, wherein the indicated values represent:
d₁ - distance between outer edges of neighbouring stents, i.e. the length of the prosthesis comprising the vein portion, the prosthesis being formed by combining the configuration of two stents and the vein section; in the embodiment shown in Fig. 6, prosthesis length is the sum of d₂ length (length of the vein section), half the length of the proximal stent and half the length of the distal stent,
d₂ - length of the vein section sutured to the stents, i.e. distance between vein section edges sutured to the stents, which is consistent with the distance between the place for attaching sutures on the proximal stent and the place for attaching sutures on the distal stent,
d₃ - distance between inner edges of neighbouring stents, i.e. length of the vein part which is not slipped onto the retaining stents and is not pressed by the stents against the wall of the vessel.

Each of the stents which constitute a prosthesis component can be provided with sutures attached on the circumference in mutually various configurations, i.e. one of the stents can comprise 3 suture sections located every 120° on the circumference of the stent, and the other stent can comprise 2 suture sections located oppositely on the circumference of the stent (at a distance of 180°).

Attachment of sutures on the stents may be constituted by any attachment which provides connection of sutures to the stent, it can be a single loop or knot made of sutures on the stent eyelet, or a multiple loop or knot. The sutures can be also attached to the stent by means of additional retaining suture sections. The suture section can be attached to the stent eyelet in the middle of its length so that, once attached, two free ends of the suture section are available for the doctor, or it can be attached on its one end so that, once attached, one end of the suture section remains free (available for the doctor). Thereby, e.g. in case of attaching three suture sections on the circumference of one stent, 6 suture ends (two ends of each of the three attached suture sections) or 3 ends (each of the three suture sections has only one suture end available, and the other one is attached to the stent) are available for suturing the implanted vein. For example, Fig. 5 shows such an attachment of suture sections that the section visible in the upper part of the stents is attached to the stent in its central part and forms two available suture ends, and the suture sections visible below are attached to the stents in the end part so that single suture ends are available for the doctor. Fig. 5 shows the attachment of suture sections in four places on the circumference of the stent (every 90°).

The stents used in the system according to the invention can be both self-expanding stents and balloon-expandable stents. As a result, the system, which constitutes a device for delivering a configuration of stents, is also adapted in terms of its structure to the said stents. The person skilled in the art is able to predict what structure the systems intended for introducing stents forming the configuration of stents according to the invention have.

The expanding stents placed in an appropriate catheter, when being introduced into a blood vessel, remain compressed in an external shielding tube of the catheter. Then, the stent is released by the operator at the target location. It expands automatically, radially and it adopts a preset shape (a stent with a shape memory is used, the stent being set with the shape during the manufacturing process), at the same time causing pressure on the walls of the vessel in which it is placed. Systems of this type are provided with means for locking the stent in an unexpanded position and means for releasing the stent to an expanded position.

Additionally, to facilitate the release of the prosthesis from the system, the system can be provided with a configuration of strings which allow pulling of the self-expanding distal stent with the vein attached.

In turn, the balloon-expandable stents, when being introduced into the blood vessel, remain clamped outside the balloon. Having introduced the catheter into the vessel, the expandable balloon is filled to a desired shape and thereby the shape is given to the stent which, having deflated the balloon, is implanted at the target location.

The stents can be made of metals and metal alloys, e.g. the stents can be made of steel, nitinol, etc. The stents can be also made of known biocompatible biodegradable (bioresorbable) polymers or made of bioresorbable steel. The stents can be additionally coated with at least one drug, e.g. an anti-inflammatory.

The sutures, which the stents are provided with, can be made of any materials known to those skilled in the art. The sutures can be absorbable or non-absorbable.

The stents can have any diameter and lengths adapted to anatomical conditions, e.g. they can have a diameter in the range of 2 to 15 mm and a length of 10 mm to 100 mm, preferably of 20 to 50 mm.

Vein sections with a length of 20 to 350 mm can be sutured to the stent. The diameter selection for the implanted vein section must take into account the diameter of the stents used. Anatomical conditions of the pathologically changed vessel determine the size of the autogenous prosthesis used. Size of the stent must be adapted to the size of the vessel, and size of the implanted vein - to the size of the retaining stents.

The configuration of two stents for attaching a vein section can be composed of identical stents or different stents, in terms of their length, width, raw material or their manufacturing techniques.

It can be also envisaged that the stents used do not have an equal diameter along their length. In the section where the stent will be coated with the vein section slipped onto it, the stent diameter can be smaller than its diameter in the section uncovered by the vein. The latter (uncovered) portion of the stent adheres, after implantation, directly to the vessel wall, where the vascular prosthesis is installed, while the second portion of the stent adheres to the vessel wall and at the same time presses the section of the implanted vein, thereby the stent diameter can be slightly smaller in this portion and include wall thickness of the implanted vein. In other words, stents in the outer sections, which are distant from the implanted vein, can have a diameter larger than in the inner sections which face each other and are intended to be applied with the section of the implanted vein. In such an embodiment of the invention, the suture sections are attached to the stent in the place where its diameter changes, i.e. on the outer end of the stent portion which has a smaller diameter.

In the above-described embodiment of the invention, structure of the expandable balloon on which the stent of a variable diameter is clamped should be adapted to the structure of the stent. For purposes of this invention, a system described in Polish Application P.387865, where a stent and a balloon of variable diameters are presented, can be adapted.

The procedure of placing the prosthesis in the patient's body is as follows. The doctor collects a vein section 6 from the patient and prepares an appropriate section by known methods. Knowing anatomical conditions present in the pathologically affected artery, a system provided with stents of suitable size and with a suitable distance between them is selected so that effective bypassing of the part of the vessel where the aneurysm is present is possible. The section of the collected vein 6 slips onto the system from the side of a flexible narrow tip 8 so that the vein is located outside the configuration of two stents 1, 3, between them, partially covering the stents. Preferably, the suture sections 5 are attached in half the length of the stents, thereby the vein section 6 is placed so that the outer halves of the stents remain uncovered. The proximal edge 6a and the distal edge 6b of the vein are sutured to the stents in half their lengths by means of the suture sections 5 attached to the stents. The doctor threads a surgical needle with free ends of the suture sections 5 and sutures the vein edge, and repeats this procedure for each available free section of the suture 5. He does not reeve the needle with suture through the stent eyelets but the suturing is performed only on the vein tissue. Superfluous suture sections are cut off. The vein section combined with two stents - the proximal stent 1 and the distal stent 3 - forms an autogenous prosthesis ready to be placed in a blood vessel to be treated. A system sheath is slipped onto the prepared prosthesis and the entire system is introduced into the blood vessel, e.g. through the femoral artery. The prosthesis is placed at the lesion so that the stents are installed above and below the aneurysm, and the implanted section of the vein is located in the section of the aneurysm occurrence. The doctor releases the prosthesis from the system by mechanically releasing the self-expanding stents (simultaneously or sequentially) or by implanting the balloon-expandable stents (simultaneously or sequentially) through inflating and deflating the balloons. Then, the catheter from the blood vessel and from the patient's body is taken out.

Reference numerals on the drawings:
1. proximal stent
2. proximal balloon
3. distal stent
4. distal balloon
5. suture section
5a. ends of suture section cut off after suturing the vein
5'. loop retaining the suture section on the stent
6. vein section
6a. proximal edge of the vein section
6b. distal edge of the vein section
7. external tube
8. tip of the system
d₁ distance between outer edges of neighbouring, proximal and distal stents,
d₂ length of the vein section sutured to the stents,
d₃ distance between inner edges of neighbouring stents.
Z lesion in the blood vessel (aneurysm)
N wall of the blood vessel

## Claims

1. An intravascular system for introducing and attaching an autogenous vascular prosthesis, comprising two stents, **characterised in that** it comprises a proximal stent **(1)** and a distal stent **(3),** located one behind the other, each of the stents being provided with at least two suture sections **(5),** attached to the stents, free ends of the said sections being located outside the lumen of the stents.

2. The system according to claim 1, **characterised in that** the proximal stent **(1)** is provided with two or three, or four suture sections **(5),** and the distal stent (3) is provided with two or three, or four suture sections **(5).**

3. The system according to claim 1, **characterised in that** the suture sections attached to one stent are located at the same distance from the edge of this stent, wherein distances of suture attachment in the proximal stent and in the distal stent can be the same or different.

4. The system according to claim 1 or 2, or 3, **characterised in that** the suture sections are attached on the circumference of the stent at equal distances to each other.

5. The system according to claim 1 or 2, or 3, or 4, **characterised in that** the suture sections are attached to the stents at a distance comprised in the range of 25% to 75% of the length of the stents.

6. The system according to claim 1 or 2, or 3, or 4, or 5, **characterised in that** the stents comprised therein are constituted by self-expanding and/or balloon-expandable stents.

7. A method of attaching an autogenous vascular prosthesis, especially at the location of aneurysm occurrence within the knee joint, **characterised in that** a vein section **(6)** collected from the patient overlaps extracorporeally the intravascular system comprising a proximal stent **(1)** and a distal stent **(3),** located one behind the other, each of them being provided with at least two suture sections **(5),** attached to the stents, so that the system is located in the lumen of the implanted vein, and then, still extracorporeally, by free ends of the suture sections located outside the lumen of the stents **(1, 3),** the proximal edge **(6a)** of the vein is sutured to the proximal stent **(1),** on its circumference, and the distal edge **(6b)** of the vein is sutured to the distal stent **(3),** on its circumference, superfluous suture sections are removed, then the system comprising the prepared autogenous prosthesis is placed in a shielding tube and is introduced intravascularly to the location of aneurysm occurrence, the prosthesis is placed in position in the section of aneurysm occurrence, and the proximal and distal stents of the prosthesis are attached sequentially or simultaneously, and the entire autogenous prosthesis is attached in the vessel by releasing it from the system.
